(19) Europäisches Patentamt
European Patent Office
Office européen des brevets

(11) Numéro de publication : **0 572 308 A2**

(12) **DEMANDE DE BREVET EUROPEEN**

(21) Numéro de dépôt : **93401329.3**

(22) Date de dépôt : **25.05.93**

(51) Int. Cl.[5] : **C07D 279/22**, C07D 279/28, C07D 279/34, C07D 417/06, C07D 498/10, C07D 279/16, C07D 277/68, C07D 277/64, C07D 417/12, A61K 31/54, A61K 31/425

(30) Priorité : **25.05.92 FR 9206353**

(43) Date de publication de la demande : **01.12.93 Bulletin 93/48**

(84) Etats contractants désignés : **AT BE CH DE DK ES FR GB GR IE IT LI LU NL PT SE**

(71) Demandeur : **ADIR ET COMPAGNIE**
**1 rue Carle Hébert**
**F-92415 Courbevoie Cédex (FR)**

(72) Inventeur : **Brion, Jean-Daniel**
**76 rue du Château**
**F-95320 Saint-Leu-La-Foret (FR)**

Inventeur : **Chollet, Anne-Marie**
**45 rue Raphael Corby**
**F-78220 Viroflay (FR)**
Inventeur : **Demuynck, Luc**
**6 rue de la Bourie Rouge**
**F-45000 Orleans (FR)**
Inventeur : **De Montarby, Lucy**
**21 rue Victor Hugo**
**F-92400 Courbevoie (FR)**
Inventeur : **Rolland, Yves**
**76 rue Jean Jaurès**
**F-92170 Vanves (FR)**
Inventeur : **Bonnet, Jacqueline**
**19 rue Charcot**
**F-75013 Paris (FR)**
Inventeur : **Ghezzi, Pietro**
**19 via Delfico Melchiorre**
**Milan (IT)**
Inventeur : **Fradin, Armel**
**10 Villa Emile Bergerat**
**F-92000 Neuilly-sur-Seine (FR)**

(54) **Dérivés hétérocycliques, leur procédé de préparation et les compositions qui les contiennent.**

(57) Composés de formule générale (I) :

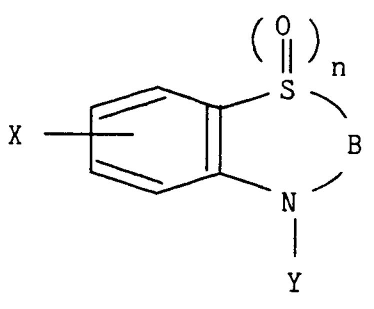

(I)

dans laquelle X, n, B et Y sont tels que définis dans la description.
Médicaments.

EP 0 572 308 A2

La présente invention concerne de nouveaux dérivés hétérocycliques, leur procédé de préparation et les compositions pharmaceutiques qui les contiennent.

Quelques dérivés hétérocycliques proches de ceux de la présente invention ont été décrits dans les brevets US-A-2519886 et US-A-2645640 pour leurs propriétés antidyspnéiques, antiasthmatiques, ganglioplégiques, spasmolytiques ou analgésiques.

Les composés de la présente invention, outre le fait qu'ils soient nouveaux, présentent une activité inhibitrice vis-à-vis des cytokines, dont le facteur nécrosant des tumeurs (TNF), et trouvent donc une application originale dans les affections pouvant induire un choc septique, les affections inflammatoires, les rejets de greffons, la douleur, l'hypertension,...

La présente invention concerne plus particulièrement les composés de formule générale (I) :

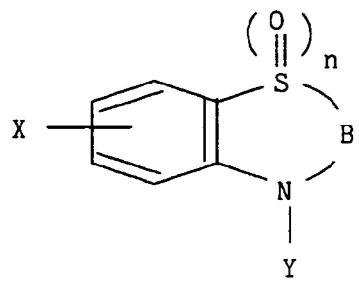

(I)

dans laquelle :

- X représente un atome d'halogène choisi parmi fluor, chlore, brome et iode,
- n représente 0, 1 ou 2,
- B représente un des groupements $B_1$, $B_2$, $B_3$ de formules :

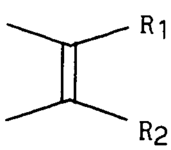

($B_1$)

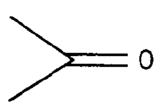

($B_2$)

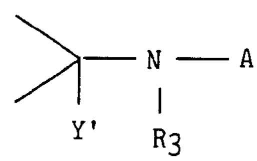

($B_3$)

dans lesquelles :

- $R_1$ et $R_2$ représentent indépendamment l'un de l'autre un atome d'hydrogène, un groupement alkyle éventuellement substitué et contenant de 1 à 5 atomes de carbone en chaîne droite ou ramifiée, un groupement aryle éventuellement substitué, un groupement arylalkyle éventuellement substitué, un groupement hétéroaryle éventuellement substitué,
  ou bien $R_1$ et $R_2$ forment ensemble un cycle aromatique contenant six atomes de carbone éventuellement substitué par un groupement X' représentant un atome d'halogène choisi parmi fluor, chlore, brome et iode,
- $R_3$ représente un atome d'hydrogène, un groupement aryle éventuellement substitué, un groupement arylalkyle éventuellement substitué, un groupement hétéroaryle éventuellement substitué,
- A représente soit :
  * le radical

$$-(SO_2)_x- \text{ Alk - Z}$$

dans lequel x représente 0 ou 1, Alk représente un radical hydrocarboné saturé et bivalent, contenant de 3 à 6 atomes de carbone en chaîne droite ou ramifiée, et Z représente un radical choisi dans le groupe comprenant :

a) le radical nitrile, carboxy ou alcoxycarbonyle

b) le radical

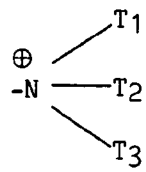

, Q⊖

dans lequel :

$T_1$, $T_2$, $T_3$, identiques ou différents, sont choisis parmi les radicaux alkyles linéaires ou ramifiés contenant de 1 à 5 atomes de carbone, les radicaux aryles éventuellement substitués et les radicaux arylalkyles éventuellement substitués,

Q représente un atome d'halogène choisi parmi chlore, brome et iode, ou un résidu d'acide faible, tel que acétate, fumarate, triflate,...

c) le radical

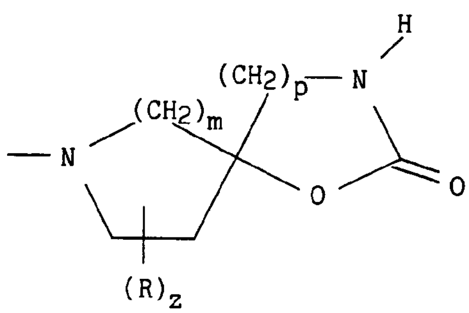

dans lequel m et p représentent indépendamment l'un de l'autre 1 ou 2, z représente un entier prenant les valeurs 0 à (m+2) (bornes incluses) et R représente un radical alkyle contenant de 1 à 5 atomes de carbone en chaîne droite ou ramifiée, un radical aryle ou arylalkyle, éventuellement substitués,

d) le radical

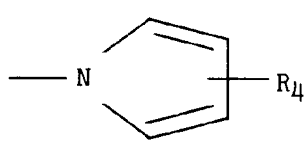

dans lequel $R_4$ représente un atome d'hydrogène, un radical carboxy, un radical alkoxycarbonyle, ou un radical aminocarbonyle éventuellement substitué,

e) le radical

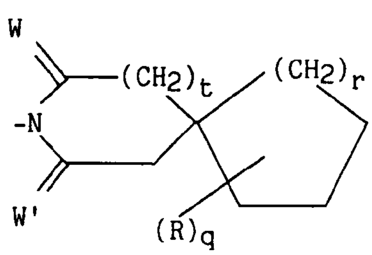

dans lequel W et W' représentent indépendamment l'un de l'autre $H_2$, O ou S, t représente 0 ou 1, r représente 1 ou 2, q représente un entier prenant les valeurs 0 à (r+2) (bornes incluses) et R a la même signification que précédemment,

f) le radical

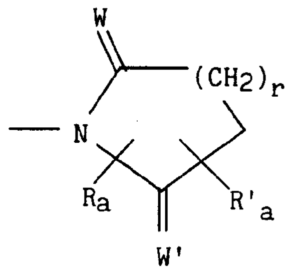

dans lequel W, W' et r sont tels que définis précédemment et $R_a$ et $R'_a$ représentent indépendamment l'un de l'autre un atome d'hydrogène, un radical alkyle contenant de 1 à 5 atomes de carbone en chaîne droite ou ramifiée, un radical aryle éventuellement substitué ou un radical arylalkyle éventuellement substitué,

g) le radical

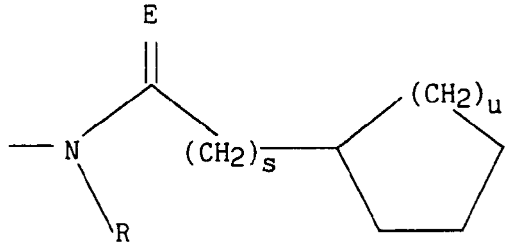

dans lequel E représente O, S ou le groupement N-H, s et u représentent indépendamment l'un de l'autre 1 ou 2 et R est tel que défini précédemment,

* soit le radical

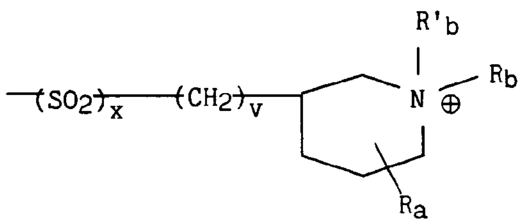

dans lequel v représente un entier prenant les valeurs 1 à 4 (bornes incluses), x et $R_a$ sont tels que définis précédemment et $R_b$ et $R'_b$ représentent indépendamment l'un de l'autre un radical alkyle contenant de 1 à 5 atomes de carbone en chaîne droite ou ramifiée un radical aryle éventuellement substitué ou un radical arylalkyle éventuellement substitué.

* soit, lorsque B représente le radical $B_3$ précédemment défini, le radical

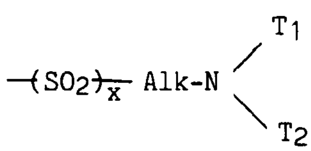

dans lequel x, Alk, $T_1$ et $T_2$ sont tels que définis précédemment,

* soit le radical

$$-(SO_2)_x- R_5$$

dans lequel x est tel que défini précédemment et $R_5$ représente un radical hydrocarboné contenant de 5 à 20 atomes de carbone en chaîne droite ou ramifiée et comprenant au moins une insaturation sous forme de double liaison,

- Y' forme avec Y une liaison,
- et Y, lorsque B représente les radicaux $B_1$ ou $B_2$ représente le groupement A tel que défini précédemment,

avec les restrictions suivantes :

- lorsque, simultanément B représente le groupement $B_2$, A représente le radical $-(SO_2)_x$Alk-Z et x est différent de 0, alors Z ne peut représenter ni un radical nitrile, ni un radical carboxy, ni un radical alcoxycarbonyle,
- lorsque, simultanément B représente le radical $B_1$ dans lequel $R_1$ et $R_2$ forment ensemble un cycle aromatique à six carbones et A représente le radical

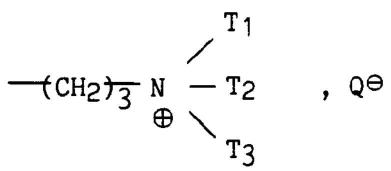

alors $Q^{\ominus}$ ne peut représenter que l'anion $Br^{\ominus}$,

étant entendu que, sauf indication contraire, le terme "radical aryle" représente un radical choisi parmi phényle et naphtyle, que le terme "radical arylalkyle" représente un radical choisi parmi phényle et naphtyle fixé sur une chaîne alkyle comportant de 1 à 4 atomes de carbone, et que le terme "radical hétéroaryle" représente un radical choisi parmi pyridyle, pyrrolyle, pyrazinyle, pyridazinyle, pyrimidinyle, quinolyle et indolyle,

étant également entendu que, sauf indication contraire, l'expression "éventuellement substitué" signifie que les groupements alkyles, aryles, arylalkyles ou hétéroaryles peuvent être substitués par un ou plusieurs groupements choisis parmi les atomes d'halogène, les groupements hydroxy, nitro, cyano, alkyles, alkoxy, acyles, halogénoalkyles, amino, aminoalkyles et diaminoalkyles,

ainsi que leurs éventuels stéréoisomères, épimères, N-oxydes et sels d'addition à un acide ou à une base pharmaceutiquement acceptables.

Parmi les acides pharmaceutiquement acceptables que l'on peut utiliser pour former un sel d'addition avec les composés de l'invention, on peut citer à titre d'exemples non limitatifs, les acides chlorhydrique, phosphorique, sulfurique, tartrique, citrique, maléique, fumarique, alkylsulfonique et camphorique.

Parmi les bases pharmaceutiquement acceptables que l'on peut utiliser pour former un sel d'addition avec les composés de l'invention, on peut citer, à titre d'exemples non limitatifs, l'hydroxyde de sodium ou de potassium, la diéthylamine, la triéthylamine, l'éthanolamine, la diéthanolamine, l'arginine et la lysine.

La présente invention a également pour objet le procédé de préparation des composés de formule (I), caractérisé en ce que le produit de départ est :

a) - soit le composé de formule (II) :

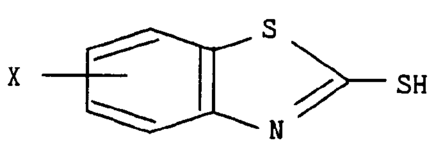

(II)

dans laquelle X est tel que défini pour la formule (I),

qui, traité par le chlorure de sulfuryle à température ambiante, conduit au composé de formule (III) :

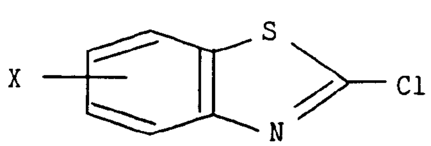

(III)

dans laquelle X est tel que défini précédemment,

puis par une amine de formule $H_2N-R_3$, dans laquelle $R_3$ est tel que défini précédemment pour donner le composé de formule (IV) :

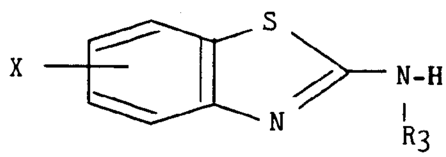

(IV)

dans laquelle X et $R_3$ sont tels que définis précédemment,

b)- soit le composé de formule (V) :

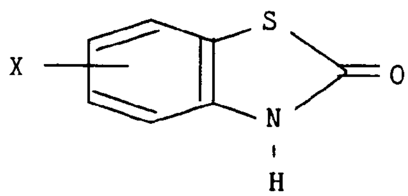

(V)

dans laquelle X est tel que défini précédemment,

c)- soit le composé de formule (VI) :

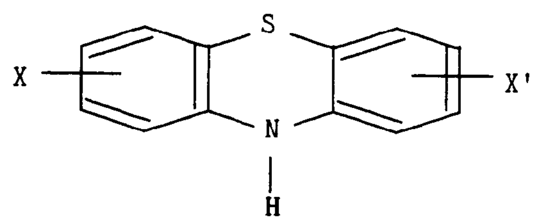

(VI)

dans laquelle X et X' sont tels que définis précédemment,

les composés de formules (IV), (V) ou (VI) formant l'ensemble des composés de formule (VII) :

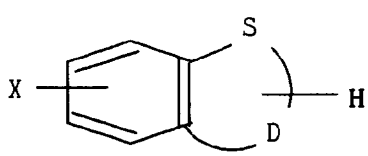

(VII)

dans laquelle X est tel que défini précédemment et D représente un des groupements $D_1$, $D_2$, ou $D_3$ de formules :

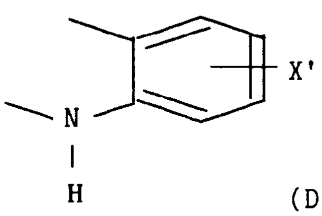

($D_1$)

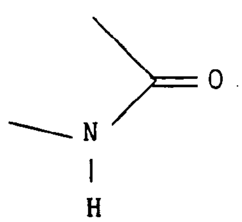

($D_2$)

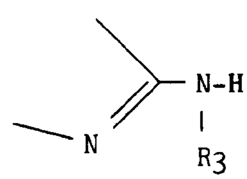

($D_3$)

dans lesquelles X' et $R_3$ sont tels que définis précédemment,

que l'on oxyde éventuellement :

- en S-monoxyde au moyen d'un réactif approprié par exemple l'acide nitrique, suivi d'une neutralisation par une solution alcaline, par exemple d'hydroxyde de sodium,
- en S-dioxyde, après protection éventuelle de l'atome d'azote, par exemple par l'anhydride acétique, avec un agent oxydant approprié, tel que l'eau oxygénée dans l'acide acétique,

de manière à obtenir les composés de formule (VIII) :

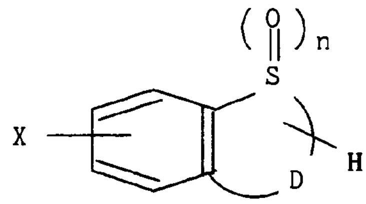

(VIII)

où X, n et D sont tels que définis précédemment,

qui sont soumis :

a) soit à une alkylation, par exemple à l'aide d'amidure de sodium, dans un solvant tel que le xylène, à reflux, avec le composé de formule (IX) :

$$Hal \leftarrow\!\!(SO_2)\!\!\rightarrow_x R_5 \qquad (IX)$$

dans laquelle x et $R_5$ sont tels que précédemment définis et Hal représente un atome d'halogène, pour obtenir les composés de formule (X) :

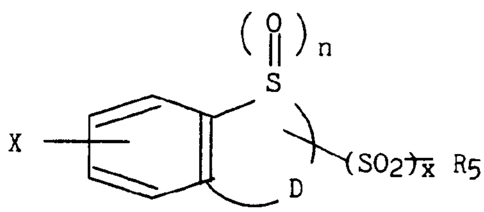

(X)

dans laquelle X, n, D, x et $R_5$ sont tels que définis plus haut,

b) soit à une alkylation, par exemple à l'aide d'amidure de sodium en solvant approprié, comme le xylène ou le dioxane, avec un composé de formule (XI) :

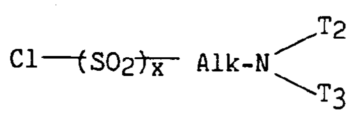

(XI)

dans laquelle x, Alk, $T_2$, et $T_3$ sont tels que définis précédemment,

pour donner le composé de formule (XII) :

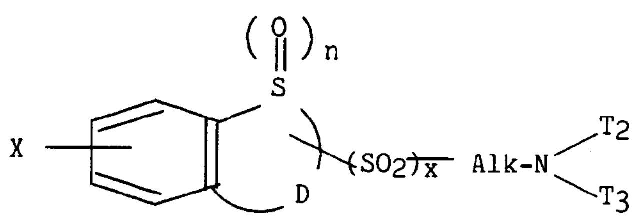

(XII)

où X, n, D, x, Alk, $T_2$ et $T_3$ ont les mêmes définitions que précédemment,

qui sera éventuellement transformé par le composé de formule (XIII) :

$$T_1\text{-}Q \qquad (XIII)$$

où $T_1$ et Q sont tels que définis précédemment, en sel d'ammonium quaternaire de formule (XIV) :

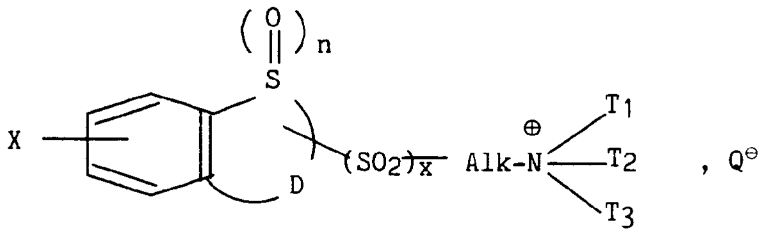

(XIV)

où X, n, D, x, Alk, $T_1$, $T_2$, $T_3$ et Q sont tels que définis précédemment,

c) soit à une alkylation, par exemple à l'aide d'hydrogénosulfate de tétrabutylammonium, dans un mélange cétone / base, par exemple méthylisobutylcétone / hydroxyde de sodium avec le composé de formule (XV) :

$$J-(SO_2)_x-\text{Alk-Cl} \qquad (XV)$$

dans laquelle x et Alk sont tels que définis précédemment et J représente un atome de chlore ou de brome,

conduisant au synthon de formule (XVI) :

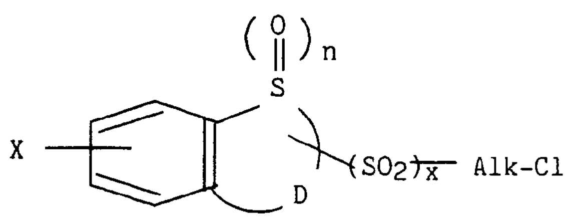

(XVI)

dans laquelle X, n, D, x et Alk sont tels que définis précédemment,

qui traité par :

α) le cyanure de tétraméthylammonium en présence d'alcool, par exemple l'isopropanol, conduit au composé de formule (XVII) :

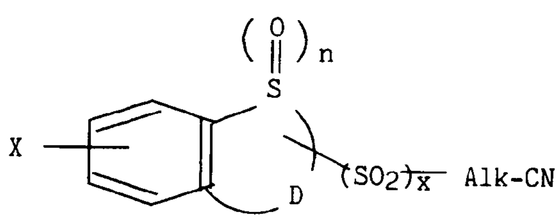

(XVII)

dans laquelle X, n, D, x et Alk sont tels que définis précédemment,

qui, par hydrolyse éventuelle, donne le composé de formule (XVIII) :

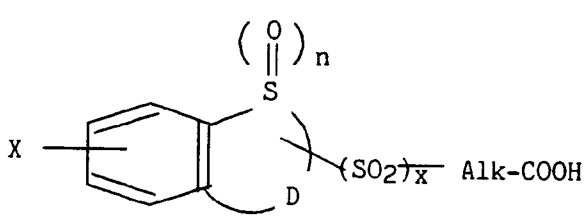

(XVIII)

dans laquelle X, n, D, x et Alk sont tels que définis précédemment,

qui peut être transformé en esters ou en amides correspondants selon les méthodes classiques,
β) un des composés suivants :

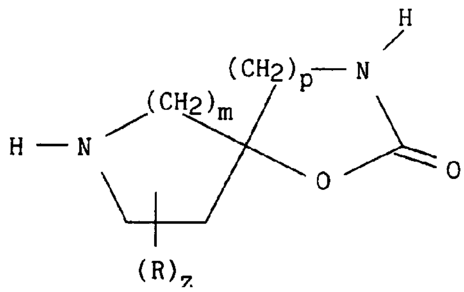

;

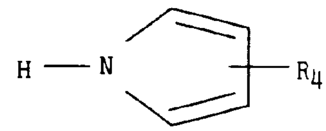

;

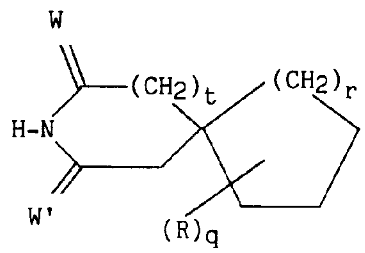

;

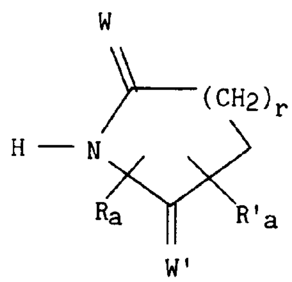

;

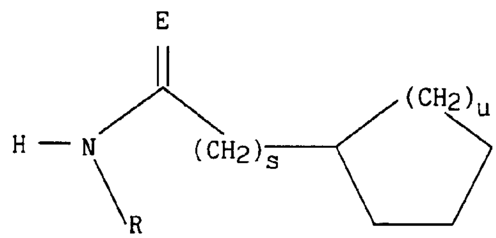

pour lesquels m, p, q, r, s, t, u, z, R, $R_a$, $R'_a$, $R_4$, E, W et W' sont tels que définis précédemment, en présence d'un sel alcalin, d'une base alcaline et d'éther couronne, conduit respectivement aux composés de formules (XIXa) à (XIXe) suivantes :

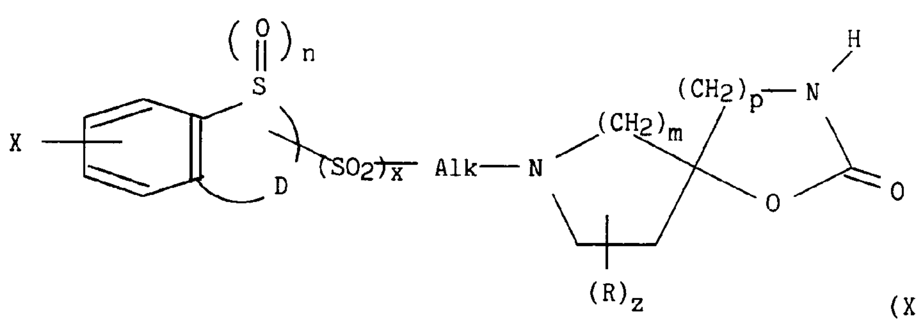

(XIXa)

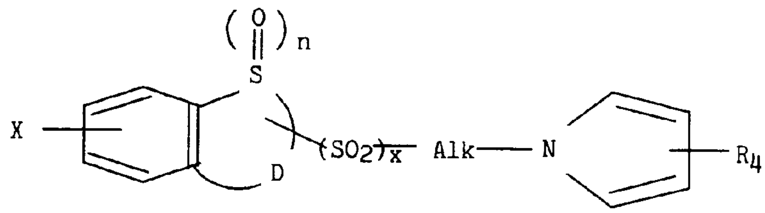

(XIXb)

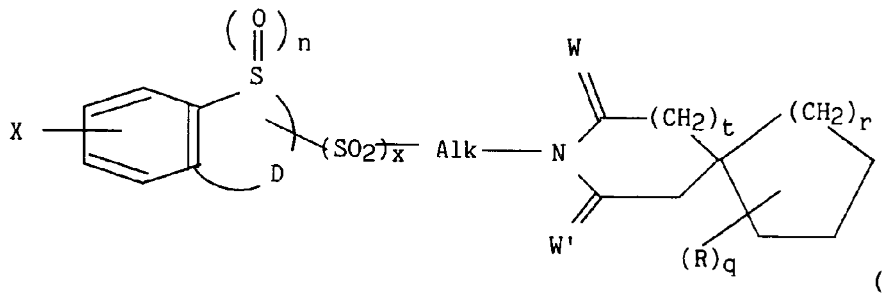

(XIXc)

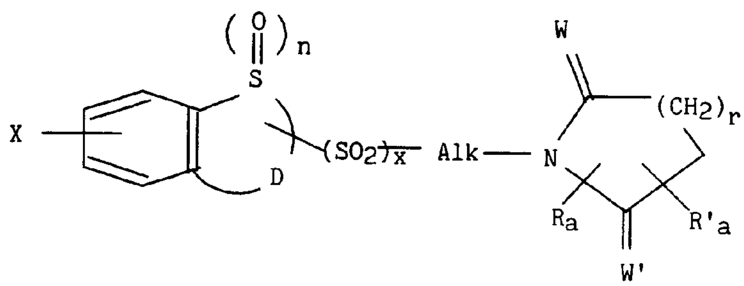

(XIXd)

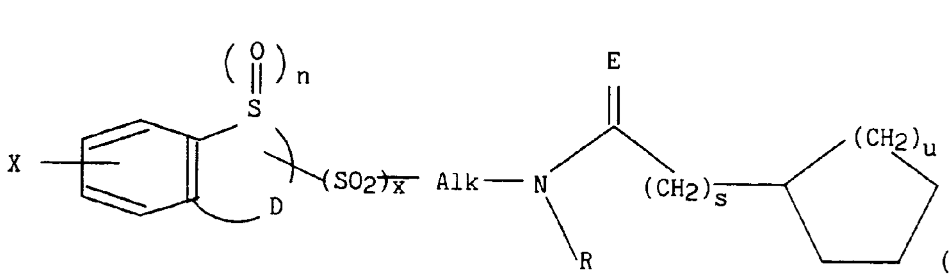

(XIXe)

dans lesquelles m, n, p, q, r, s, t, u, x, z, Alk, D, E, R, $R_a$, $R'_a$, $R_4$, W, W' et X sont tels que définis précédemment,

d) soit à une alkylation, par exemple à l'aide d'hydrogénosulfate de tétrabutylammonium, dans un mélange cétone / base, par exemple méthylisobutylcétone / hydroxyde de sodium avec le composé de formule (XX) :

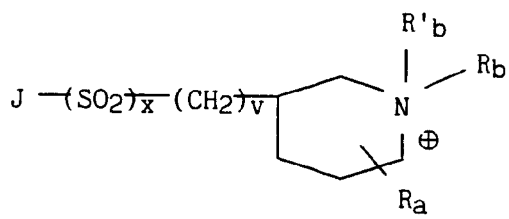

(XX)

dans laquelle J, $R_a$, $R_b$, R'b, x et v sont tels que définis précédemment,
conduisant au composé de formule (XXI) :

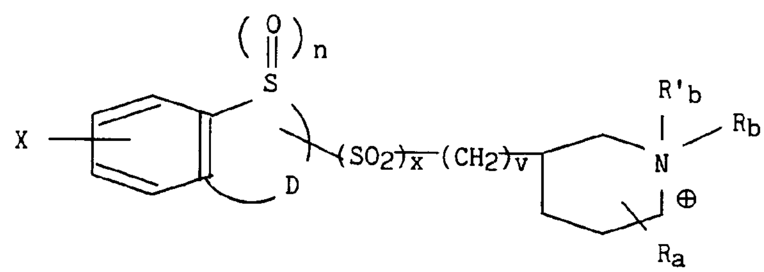

(XXI)

dans laquelle X, n, D, x, v, $R_a$, $R_b$ et $R'_b$ sont tels que définis précédemment,
les composés de formules (X), (XII), (XVII), (XIXa) à (XIXe) ou (XX) lorsque D représente le groupement

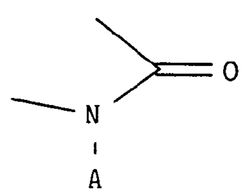
,

dans lequel A est tel défini précédement, pouvant être soumis à l'action d'une base dans un solvant alcool, par exemple dans l'hydroxyde de potassium dans l'éthanol, puis acidification, par exemple à l'aide d'acide acétique, pour conduire au composé de formule (XXII) :

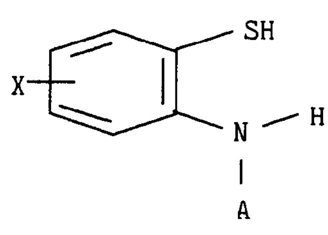

(XXII)

dans laquelle X et A sont tels que définis précédemment,
qui, traité en milieu alcalin, par exemple hydroxyde de potassium dans l'éthanol, par un composé de formule (XXIII) :

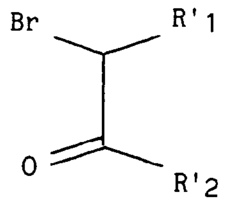

(XXIII)

dans lequel R'$_1$ et R'$_2$ représentent indépendamment l'un de l'autre un atome d'hydrogène, un groupement alkyle éventuellement substitué et contenant de 1 à 5 atomes de carbone en chaîne droite ou ramifiée, un groupement aryle éventuellement substitué ou un groupement hétéroaryle éventuellement substitué,
conduit au composé de formule (XXIV) :

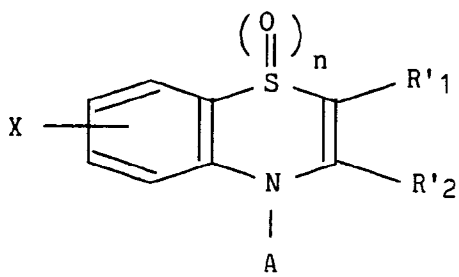

(XXIV)

dans laquelle X, A, n, R'$_1$ et R'$_2$ sont tels que définis précédemment,
l'ensemble des composés de formules (X), (XII), (XIV), (XVII), (XVIII), (XIXa) à (XIXe), (XX), (XXI) et (XXIV) formant l'ensemble des composés de formule (I) que l'on purifie le cas échéant selon une technique classique de purification et dont on sépare, si on le souhaite, les stéréoisomères par une technique classique de séparation et que l'on transforme, si nécessaire, en leurs sels d'addition à un acide ou une base pharmaceutiquement acceptables, composés qui, lorsque n = 0 peuvent être oxydés en S-oxyde ou S-dioxyde correspondants.

Les composés de formule (I) possèdent des propriétés pharmacologiques nouvelles. Administrés à la souris, ils inhibent la production du facteur nécrosant des tumeurs (TNF) impliqué dans un grand nombre de situations pathologiques allant de l'inflammation au choc septique.

Le TNF est une cytokine secrétée après activation de macrophages, de monocytes ou d'autres cellules immunocompétentes et qui, une fois libérée dans la circulation et les tissus, se fixe sur des récepteurs spécifiques. Il joue, avec l'interleukine-1 (IL-1), un rôle déterminant dans la survenue d'effets inflammatoires et métaboliques. Les effets biologiques et/ou pathologiques sont multiples : fièvre, fuite capillaire, coagulation intravasculaire, inflammation, perte protéique, cachexie...

Les exemples suivants illustrent l'invention sans la limiter d'aucune façon. Les produits de départ sont connus ou préparés à partir de modes opératoires connus.

**Préparation A : Préparation de 2-chloro-10-(3-diméthylaminopropyl)phénothiazine**

3,56 g (10 mmoles) de chlorhydrate de 2-chloro-10-(3-diméthylaminopropyl)phénothiazine sont mis en suspension dans 15 ml d'une solution d'hydroxyde de sodium 1N. L'amine libérée est extraite à l'éther éthylique. La phase organique décantée est séchée sur sulfate de sodium puis évaporée à sec.3,13 g d'une huile correspondant au produit attendu sont obtenus.
Rendement : 98 %

**Préparation B : Nitrate de 2-chloro-10-(3-diméthylaminopropyl)phénothiazine-5-oxyde**

Une solution de 3,56 g (10 mmoles) de chlorhydrate de 2-chloro-10-(3-diméthylaminopropyl)phénothiazine dans 50 ml d'eau est additionnée goutte à goutte à 5,5 ml d'une solution d'acide nitrique 11N. Le mélange est maintenu sous agitation à température ambiante pendant 30 minutes. Le pH est ensuite amené à une valeur de 3-4 par addition d'une solution d'hydroxyde de sodium 1N. Après 15 heures d'agitation à 0°C, le produit attendu cristallise. Après filtration et séchage sous vide phosphorique, 3,7 g du produit attendu sont obtenus.
Rendement : 93 %

Point de fusion : 102-104°C

**Préparation C : 2-Chloro-10-(3-diméthylaminopropyl)phénothiazine-5-dioxyde**

Etape a/ 2-Chloro-10-acétylphénothiazine-5-dioxyde

4,2 g (15 mmoles) de 2-chloro-10-acétylphénothiazine sont mis en solution dans 30 ml d'acide acétique glacial. 3,25 ml d'une solution de peroxyde d'hydrogène (à 20 % dans l'eau) sont ajoutés. Le mélange est porté à 90°C pendant 4 heures 30. Après retour à température ambiante, l'addition d'eau provoque la précipitation du produit attendu. Après filtration, lavage à l'eau et séchage sous vide phosphorique, 4 g de sulfone sont obtenus.
Rendement : 87 %
Point de fusion : 195-197°C

Etape b/ 2-Chlorophénothiazine-5-dioxyde

La sulfone obtenue dans l'étape a/ est mise sous agitation dans 100 ml d'un mélange 1:1 d'hydroxyde de sodium 1N et d'éthanol. Après deux heures de réaction à reflux, l'éthanol est évaporé. Le précipité est filtré, lavé à l'eau puis séché sous vide phosphorique.
Rendement : 96 %
Point de fusion : 271-273°C

Etape c/ 2-Chloro-10-(3-diméthylaminopropyl)phénothiazine-5-dioxyde

A 5,3 g (20 mmoles) de la sulfone obtenue précédemment dans 200 ml de dioxane est ajouté 1,17 g (30 mmoles) d'amidure de sodium. Le mélange est porté à reflux pendant 3 heures. 4,9 g (40 mmoles) de 3-chloro-N,N-diméthylpropanamine dans 20 ml de toluène sont additionnés à la solution précédente. Après 20 heures de reflux, le solide formé est éliminé par filtration. 3 g de produit sont obtenus après évaporation du solvant et recristallisation dans l'éthanol.
Rendement : 60 %
Point de fusion : 105-106°C

**Préparation D : 2-Chloro-10-(3-chloropropyl)phénothiazine**

Une solution de 2,34 g (10 mmoles) de 2-chlorophénothiazine, 4,73 g (30 mmoles) de 1-bromo-3-chloropropane et de 0,34 g (1 mmol) d'hydrogénosulfate de tétrabutylammonium dans 40 ml de 4-méthylpentan-2-one est additionnée de 40 ml d'une solution d'hydroxyde de sodium (50 % en poids). Le mélange est agité à température ambiante pendant 48 heures. Après traitement habituel de la phase organique, séchage sur sulfate de sodium, évaporation du solvant et purification par chromatographie sur gel de silice, 2,17 g de produit pur sont obtenus (Eluant : éther de pétrole).
Rendement : 70 %
Point de fusion : 51-52°C

**Preparation E : 3-(3-Diméthylammoniopropyl)-5-chlorobenzothiazolin-2-one**

Composé préparé selon le mode opératoire décrit à la préparation C, étape c), à partir de 5-chlorobenzothiazolin-2-one.
Rendement : 87 %
Point de fusion : 206-208°C

**Exemple 1 : Bromure de N-[3-(2-chlorophénothiazin-10-yl)propyl]triméthylammonium**

A 3,2 g (10 mmoles) d'huile obtenue dans la préparation A sont ajoutés à 0°C et sous atmosphère d'argon, 150 mmoles de bromure de méthyle (solution 2M dans l'éther éthylique). Après 8 à 12 heures d'agitation à température ambiante, le composé formé est séparé par filtration, lavé à l'éther éthylique et séché sous vide phosphorique. 2,7 g de produit attendu sont obtenus.
Rendement : 65 %
Point de fusion : 137-140°C

**Exemple 2 : Bromure de N-[3-(2-chloro-5-oxo-phénothiazin-10-yl)propyl]triméthylammonium**

Le nitrate obtenu dans l'exemple B est traité de manière analogue à celle décrite dans la préparation A. L'amine libre ainsi obtenue est méthylée selon la méthode décrite dans l'exemple 1.
Rendement : 95 %
Point de fusion : 240-243°C

**Exemple 3 : Bromure de N-[3-(2-chloro-5-dioxo-phénothiazin-10-yl)propyl]triméthylammonium**

Ce composé est obtenu selon le mode opératoire décrit dans l'exemple 1 en utilisant comme produit de départ la sulfone de la préparation C.
Rendement : 73 %
Point de fusion : 183-184°C

**Exemple 4 : Bromure de N-[3-(2-chlorophénothiazin-10-yl)propyl]diméthyléthylammonium**

Synthèse identique à celle décrite dans l'exemple 1, l'agent alkylant étant cette fois-ci le bromure d'éthyle.
Rendement : 88 %
Point de fusion : 176-177°C

**Exemple 5 : Bromure de N-[3-(2-chlorophénothiazin-10-yl)propyl]diméthylpropylammonium**

Même remarque que pour l'exemple 4, l'alkylation étant réalisée ici par le bromure de propyle.
Rendement : 66 %
Point de fusion : 213-215°C

**Exemple 6 : Bromure de N-[3-(2-chlorophénothiazin-10-yl)propyl]diméthylisopropylammonium**

Synthèse identique à celle de l'exemple 5 en utilisant le bromure d'isopropyle.
Rendement : 61 %
Point de fusion : 153-154 °C

**Exemple 7 : Bromure de N-[3-(2-chlorophénothiazin-10-yl)propyl]butyldiméthylammonium**

Même remarque que pour l'exemple 4, l'agent alkylant étant ici le bromure de n-butyle.
Rendement : 68 %
Point de fusion : 191°C

**Exemple 8 : Bromure de N-[3-(2-chloro-5-oxo-phénothiazin-10-yl)propyl]butyldiméthylammonium**

Synthèse analogue à celle décrite dans l'exemple 2, l'agent alkylant étant dans ce cas le bromure de n-butyle.
Rendement : 81 %
Point de fusion : 260-264°C

**Exemple 9 : Bromure de N-[3-(2-chloro-5-dioxo-phénothiazin-10-yl)propyl]butyldiméthylammonium**

Une solution de 1,75 g (5 mmoles) de sulfone obtenue dans la préparation C, dans 20 ml de 1-bromobutane est portée à reflux pendant 8 heures, puis laissée sous agitation à température ambiante pendant 12 heures. Le précipité obtenu est filtré, puis lavé abondamment à l'éther éthylique. Après purification par recristallisation dans un mélange méthanol/éthanol, 1,58 g de produit attendu est obtenu.
Rendement : 65 %
Point de fusion : 270-274°C

**Exemple 10 : Bromure de N-[3-(2-chlorophénothiazin-10-yl)propyl]benzyldiméthylammonium**

3,2 g (10 mmoles) du composé de la préparation A sont mis en solution dans 35 ml de tétrahydrofurane, auxquels sont ajoutés ensuite 3,18 g (20 mmoles) de bromure de benzyle en solution dans le tétrahydrofurane.

Le mélange réactionnel est porté à reflux pendant 15 heures. Le précipité formé, séparé par filtration, lavé à l'éther éthylique puis séché sous vide phosphorique, fournit 2,84 g de produit attendu.
Rendement : 58 %
Point de fusion : 140-142°C

**Exemple 11 : 1-[3-(2-Chlorophénothiazin-10-yl)-1-propyl]pyrrole-2-carboxylate de méthyle**

Sous atmosphère d'argon sont introduits successivement 3,17 g (25 mmoles) de pyrrole-2-carboxylate de méthyle, 2,07 g (15 mmoles) de carbonate de potassium, 1,54 g (27,5 mmoles) d'hydroxyde de potassium en poudre et 0,66 g (2,5 mmoles) d'éther 18-couronne-6, en suspension dans 120 ml de toluène. Une agitation pendant 2 heures à température ambiante provoque la précipitation de l'anion pyrrolique. 7,75 g (25 mmoles) de composé obtenu dans la préparation D dans 25 ml de toluène sont ajoutés goutte à goutte. Le mélange est ensuite porté six heures à reflux. Après hydrolyse par une solution saturée de chlorure de potassium, extraction à l'éther éthylique et lavage à l'eau des phases organiques réunies, celles-ci sont séchées sur sulfate de sodium et le solvant est évaporé.
Après purification par chromatographie sur gel de silice, 5,98 g de produit attendu sont obtenus (Eluant : dichlorométhane/cyclohexane).
Rendement : 60 %
Point de fusion : 90-92°C

**Exemple 12 : Acide 1-[3-(2-chlorophénothiazin-10-yl)-1-propyl]pyrrole-2-carboxylique**

3,99 g (10 mmoles) de composé obtenu dans l'exemple 11 sont mis en solution dans 15 ml d'éthanol. 0,8 g (20 mmoles) d'hydroxyde de sodium (en solution 1N) est ajouté et le mélange est porté à reflux sous agitation pendant 7 heures. Après évaporation du solvant, reprise par de l'eau, acidification par une solution 1N d'acide chlorhydrique et traitement habituel, 3,62 g de produit attendu sont obtenus.
Rendement : 94 %
Point de fusion : 162-163°C

**Exemple 13 : 8-[3-(2-Chlorophénothiazin-10-yl)-1-propyl]-2-oxo-3,8-diaza-1-oxa-spiro[4.5]décan-2-one**

Réaction identique à celle décrite dans l'exemple 11 en condensant la 3,8- diaza-1-oxa-spirodécan-2-one (composé décrit dans US-A-3594386).6,01 g de produit sont obtenus après recristallisation dans l'acétate d'éthyle.
Rendement : 56 %
Point de fusion : 142-143°C

**Exemple 14 : 2-Chloro-10-[1-(4-cyanopropyl)]phénothiazine**

A 2,48 g (8 mmoles) du composé de l'exemple D en solution dans 25 ml d'isopropanol est ajouté 1,7 g (12 mmoles) de cyanure de tétraéthylammonium dans 12 ml d'isopropanol. Le mélange est porté à reflux pendant 72 heures. Après évaporation de l'isopropanol, le chlorure de tétraéthylammonium est précipité par l'éther éthylique et éliminé par filtration. Une purification par chromatographie sur gel de silice (éluant cyclohexane/acétate d'éthyle) conduit à 2,10 g de produit attendu.
Rendement : 87 %
Point de fusion : 75-77°C

**Exemple 15 : Acide 4-(2-chlorophénothiazin-10-yl)butanoïque**

2,10 g (7 mmoles) de produit de l'exemple 14 sont mis en solution dans 20 ml d'éthanol. 1,57 g (28 mmoles) d'hydroxyde de potassium en solution dans 10 ml d'eau est ajouté et le mélange est porté à reflux pendant 12 heures, puis abandonné une nuit à température ambiante. Le milieu est alors acidifié par une solution d'acide chlorhydrique 1N. Le traitement de la phase organique par le dichlorométhane puis l'acétone fournit 1,17 g d'acide.
Rendement : 52 %
Point de fusion : 142-144°C

**Exemple 16 : Iodure de N-[3-(5-chloro-2-oxobenzothiazolin-3-yl)propyl]triméthylammonium**

Composé préparé à partir du produit obtenu dans l'exemple E, selon le mode opératoire décrit à l'exemple 1.
Rendement : 56 %
Point de fusion : 202-204°C

**Exemple 17 : 1-[3-(5-Chloro-2-oxo-benzothiazolin-3-yl)-1-propyl]pyrrole-2-carboxylate de méthyle**

Composé préparé selon les modes opératoires décrits à la préparation D puis à l'exemple 11 à partir de la 5-chlorobenzothiazolin-2-one.
Rendement : 40 %
Point de fusion : 90-91°C

**Exemple 18 : Acide 1-[3-(5-chloro-2-oxo-benzothiazolin-3-yl)-1-propyl]pyrrole-2-carboxylique**

Composé préparé suivant le mode opératoire décrit à l'exemple 12 à partir du produit décrit dans l'exemple précédent.
Rendement : 57 %
Point de fusion : 162-166°C

**Exemple 19 : 5-Chloro-3-[3-(8,8-diméthyl-1,3-dioxo-2-azaspiro[4.5]décan-2-yl)propyl]benzothiazolin-2-one**

Etape a/ 3-(3-Azidopropyl)-5-chlorobenzothiazolin-2-one

2,62 g (10 mmoles) de 3-(3-chloropropyl)-5-chlorobenzothiazolin-2-one préparé selon les modes opératoires décrits à la préparation D à partir de la 5-chlorobenzothiazolin-2-one, 0,98 g (15 mmoles) d'azoture de sodium et une quantité catalytique d'iodure de potassium sont dissous dans 40 ml de diméthylsulfoxyde. La solution est portée à 55°C sous agitation pendant 25 heures. Le traitement habituel de la phase organique conduit au produit attendu.
Rendement : 100 %

Etape b/ 3-3(Aminopropyl)-5-chlorobenzothiazolin-2-one

0,46 g (12 mmoles) d'hydrure double de lithium et d'aluminium sont mis en suspension sous argon à 0°C dans 12 ml de tétrahydrofurane anhydre. 2,15 g (8 mmoles) du composé obtenu dans l'étape a/ sont ajoutés. Le mélange est hydrolysé, la phase organique lavée avec un mélange dichlorométhane/méthanol pour donner le produit attendu.
Rendement : 71 %

Etape c/ 5-Chloro-3-[3-(8,8-diméthyl-1,3-dioxo-2-azaspiro[4.5]décan-2-yl)propyl]benzothiazolin-2-one

Un mélange équimoléculaire du composé obtenu dans l'étape b/ et de 8,8- diméthyl-2-oxaspiro[4.5]décan-1,3-dione dans le toluène est porté à reflux en présence de tamis moléculaire pendant 4 jours. Après élimination du tamis moléculaire et évaporation du solvant, le produit attendu est obtenu.
Rendement : 35 %
Point de fusion : 159-160°C

**Exemple 20 : 2-[3-(2-Chlorophénothiazin-10-yl)-1-propyl]-7,7,9,9-tétraméthyl-2-azaspiro[4.5]décan-1,3-dione**

Composé préparé de manière identique au composé de l'exemple 19.

**Exemple 21: 2-[3-(2-Chlorophénothiazin-10-yl)-1-propyl]-7,9-cis-diméthyl-2-azaspiro[4.5]décan-1,3-dione**

Composé préparé de manière identique au composé de l'exemple 19.

**Exemple 22 : Bromure de N-{3-[(2-chlorophénothiazin-10-yl)sulfonyl]propyl}triméthylammonium**

Etape a/ 2-Chloro-10-[(3-chloropropyl)sulfonyl]phénothiazine

Un mélange de 11,68 g (50 mmoles) de 2-chlorophénothiazine et de 2,34 g (60 mmoles) d'amidure de sodium dans 100 ml de xylène est porté à reflux pendant 1 à 2 heures. 50 ml de chlorure de 3-chloropropylsulfonyle dans 25 ml de xylène sont ajoutés, puis le mélange est à nouveau porté à reflux pendant une nuit. Après refroidissement du milieu réactionnel et filtration du résidu solide, le filtrat est évaporé à sec. Après distillation sous pression réduite, une huile correspondant au produit titre est obtenue.

Etape b/ 2-Chloro-10-[(3-diméthylaminopropyl)sulfonyl]phénothiazine

Le dérivé chloré obtenu à l'étape a/ (2 g) est mis en solution dans 45 ml de diméthylamine, dans un autoclave, à température ambiante. Après 24 heures de réaction, l'amine est évaporée à température ambiante et le résidu solide repris à l'éther.

Les sels d'ammonium sont précipités sous forme de chlorhydrate par ajout d'une solution éthérée d'acide chlorhydrique. Après filtration, le solide est repris par une solution 1N d'hydroxyde de sodium et extrait à l'éther. Le séchage et l'évaporation de la phase organique conduit à 1,75 g du produit attendu.

Etape c/ Bromure de N-[3-(2-chlorophénothiazin-10-yl)sulfonylpropyl]triméthylammonium

L'ammonium quaternaire est formé à partir de l'amine obtenue à l'étape b/ en utilisant les conditions de réaction précisées dans l'exemple 1.
Rendement : 40 %
Point de fusion : 216-217°C

**Exemple 23 : Bromure de N-[3-(6-chloro-3-phényl-4H-1,4-benzothiazin-4-yl)propyl]triméthylammonium**

Etape a/ 4-Chloro-2-(3,3-diméthylaminopropyl)aminothiophénol

A 11,35 g du composé obtenu dans la préparation E (41,9 mmol) dans 50 ml d'éthanol sont ajoutés 9,4 g (167,7 mmol) d'hydroxyde de potassium. Le mélange est porté à reflux pendant 2 heures puis le solvant est évaporé.

Le résidu huileux est repris par 100 ml d'eau, lavé par du toluène puis acidifié par de l'acide acétique à 10 %. L'huile cristallise ensuite pour donner, après filtration, 6,2 g du produit attendu.
Rendement : 60 %.

Etape b/ 4-(3,3-Diméthylaminopropyl)-3-phényl-6-chlorobenzothiazine

A une solution de 1,68 g d'hydroxyde de potassium (30 mmol), dans 45 ml d'éthanol sont ajoutés 7,34 g (30 mmol) du composé obtenu à l'étape a/ sous argon. 6,86 g (34,5 mmol) de bromoacétophénone en solution dans 80 ml d'éthanol sont additionnés ensuite goutte à goutte. Le mélange est porté à reflux pendant 20 heures.

Après évaporation du solvant, le milieu est repris par de l'acide acétique à 10 %, lavé à l'éther et alcalinisé par une solution concentrée d'hydroxyde de sodium. L'extraction à l'éther de cette phase aqueuse basique conduit après séchage sur sulfate de sodium, évaporation et purification par chromatographie sur gel de silice, à 5,6 g de 1-{[4-chloro-2-(3,3-diméthylpropyl)amino]phénylthio}acétophénone. Ceux-ci, en solution de le toluène, en présence du complexe trifluorure de bore/éther éthylique, porté à reflux pendant 2 heures 20, conduisent, après traitement habituel à 1,8 g du composé attendu.
Rendement : 34 %

Etape c/ Bromure de N-[3-(6-chloro-3-phényl-4H-1,4-benzothiazin-4-yl)propyl]triméthylammonium

0,53 g du composé obtenu à l'étape b/ est traité de manière analogue à celle décrite dans l'exemple 1 pour conduire à 0,48 g d'ammonium quaternaire.
Rendement : 72 %
Point de fusion : 225-226°C

**Exemple 24 : Dichlorure de 2-(3-diméthylammoniopropylamino)-5-chlorobenzothiazolium**

Etape a/ 2,5-Dichlorobenzothiazole

9,15 g de 5-chloro-2-mercaptobenzothiazole sont mis à réagir dans 50 ml de chlorure de sulfuryle à température ambiante pendant 1 heure 30. Après hydrolyse, l'extraction au dichlorométhane de la phase aqueuse et le traitement habituel de la phase organique conduisent à 8,3 g de produit attendu.
Rendement : 90 %
Point de fusion : 63-64°C

Etape b/ 2-[(3-Diméthylaminopropyl)amino]-5-chlorobenzothiazole

Composé obtenu selon le mode opératoire décrit dans l'exemple 11 en utilisant le 3,5-dichlorobenzothiazole et la N,N-diméthylaminopropylamine.
Rendement : 70 %

Etape c/ Dichlorure de 2-(3-diméthylammoniopropylamino)-5-chlorobenzothiazolium

Le composé obtenu à l'étape b/ est repris par de l'éthanol. Le chlorhydrate est formé par ajout d'une solution saturée d'acide chlorhydrique dans l'éthanol.
Rendement : 90 %
Point de fusion : 275-276°C

**Exemple 25 : Trichlorure de N-(3-diméthylammonio-1-propyl)-N-(3-pyridiniométhyl)-2-amino-5-chlorobenzothiazolium**

La condensation de 0,6 g de N,N-diméthyl-3-[(pyrid-3-yl)méthylamino] propylamine (obtenue par hydrogénation du produit de réaction de la 2-formylpyridine sur la 3-(N,N-diméthylamino)propylamine) avec 0,7 g de 2,5-dichlorobenzothiazole conduit à l'amine tertiaire attendue selon le mode opératoire décrit à l'exemple 11. Le chlorhydrate est alors formé en reprenant le produit brut par une solution d'acide chlorhydrique dans l'éthanol. L'addition d'éther au milieu précipite 0,45 g de composé trisalifié.
Rendement : 34 %
Point de fusion : 236-238°C

**Exemple 26 : 2-Chloro-10-[(2*E*)-1-(3,7-diméthylocta-2,6-diényl)]phénothiazine**

Composé obtenu, sous forme d'huile, selon le mode opératoire décrit dans la préparation C, étape c/, à partir de 2-chlorophénothiazine et de (2*E*)-1-chloro-3,7-diméthylocta-2,6-diène.
Rendement : 20 %

**Exemple 27 : Chlorure de 2-[3-(2-chlorophénothiazin-10-yl)propyl]-8-tertiobutyl-2-azoniaspiro[4.5]décane**

Composé préparé de manière identique au composé de l'exemple 19.
Point de fusion : 227-228°C

**Exemple 28 : Chlorure de 2-[3-(2-chlorophénothiazin-10-yl)propyl]-8,8-diméthyl-2-azoniaspiro[4.5]décane**

Composé préparé de manière identique au composé de l'exemple 19.
Point de fusion : 100-105°C

**Exemple 29 : Dibromure de [N-(5-chlorobenzothiazol-2-yl)ammonio]propyl-triméthylammonium.**

L'ammonium quaternaire est préparé à partir du composé de l'exemple 24 suivant le mode opératoire décrit dans l'exemple 1. Celui-ci est ensuite repris par de l'éthanol et traité par une solution saturée d'acide bromhydrique dans l'éthanol. Le bromhydrate ainsi formé est isolé par filtration.

**Exemple 30 : Chlorure de bis-(5-chlorobenzothiazol-2-yl)-3-diméthylaminopropyl)ammonium**

Composé obtenu selon le mode opératoire décrit pour l'exemple 11 en utilisant 2 équivalents de 3,5-dichlorobenzothiazole et un équivalent de N,N-diméthylaminopropylamine.

Le chlorhydrate est ensuite préparé suivant la méthode décrite à l'exemple 24, étape c/.

Rendement : 10 %

Point de fusion : > 250°C

## ETUDE PHARMACOLOGIQUE

**Exemple 31 : Inhibition de la production de TNF**

Le test est réalisé chez la souris. Trente minutes après l'administration intrapéritonéale ou orale de la molécule à tester, les souris sont traitées par 2,5 µg de LPS provenant d'*Escherichia coli* par voie intrapéritonéale. Le taux de TNF est mesuré dans le sérum après 1 heure, par une méthode biologique.

**Tableau I**

| **Inhibition de la production de TNF** (par rapport au lot témoin ayant reçu uniquement LPS) | | |
|---|---|---|
| Composé | % inhibition après adm. (dose) | |
| | voie i.p. | voie orale |
| Exemple 1 | 75 % (5 mg/kg) | 68 % (50 mg/kg) |
| Exemple 2 | 50 % (5 mg/kg) | 60 % (50 mg/kg) |
| Exemple 3 | 69 % (5 mg/kg) | - |
| Exemple 6 | - | 38 % (50 mg/kg) |
| Exemple 7 | 95 % (5 mg/kg) | 72 % (50 mg/kg) |
| Exemple 8 | 71 % (5 mg/kg) | 73 % (50 mg/kg) |
| Exemple 9 | 75 % (5 mg/kg) | - |
| Exemple 10 | 27 % (5 mg/kg) | - |
| Exemple 15 | - | 55 % (50 mg/kg) |
| Exemple 26 | - | 55 % (50 mg/kg) |
| Exemple 27 | - | 26 % (50 mg/kg) |
| Chlorpromazine | 100 % (4 mg/kg) | - |

Les composés ici décrits diminuent nettement les taux sériques de TNF et améliorent pour certains très nettement la survie de la souris chez laquelle a été administrée préalablement par voie intra-péritonéale de l'endotoxine (ou lipopolysaccharide (LPS)).

**Exemple 32 : Effet protecteur des composés vis-à-vis du choc au LPS**

Le test est réalisé chez la souris. Le composé à tester est administré par voie intrapéritonéale ou par voie orale. Dans le premier protocole (voie ip), le composé est administré 30 minutes avant l'injection de 1 µg de LPS chez la souris. La mortalité est déterminée 72 heures après.

**Tableau II**

| % Survie des souris après administration de LPS | |
|---|---|
| Composé | % survie 72 h après administration ip |
| Exemple 1 | 80 % (5 mg/kg) |
| Exemple 7 | 40 % (5 mg/kg) |
| Exemple 10 | 60 % (5 mg/kg) |
| | 36 % (1 mg/kg) |
| Chlorpromazine | 77 % (4 mg/kg) |

**Exemple 33 : Effet périphérique des composés**

Par rapport à la chlorpromazine, les composés de l'invention ne possèdent qu'un effet périphérique. A titre d'exemple, le composé de l'exemple 1 administré en ip (8 mg/kg) au rat, ayant reçu ensuite une injection intracérébroventriculaire de LPS, diminue chez l'animal, comme la chlorpromazine, le taux sérique de TNF mais non son taux cérébral comme il est observé chez le rat traité par la chlorpromazine (Figure I).

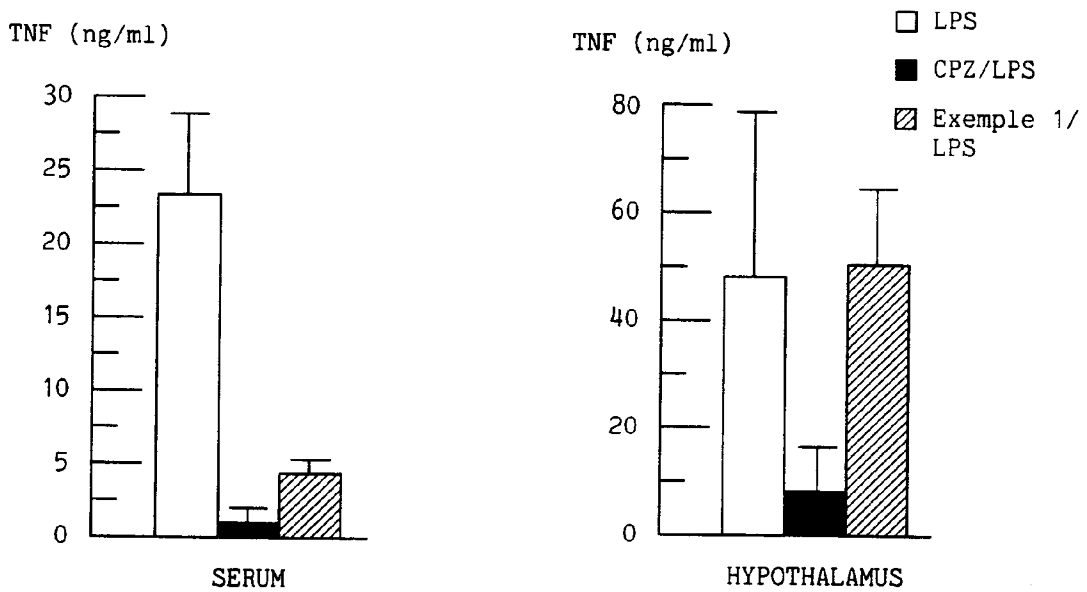


**Exemple 34 : Activité locomotrice de la souris**

Ce test consiste à déterminer l'activité motrice spontanée de la souris placée dans une enceinte constituant un environnement nouveau et équipée d'un système de rayons infra-rouges dont l'interruption lors du passage de l'animal sert de critère de mesure ("Activity monitor" Digiscan-Omnitech Electronics, Sufraco France).

Les molécules sont administrées par voie intrapéritonéale 30 minutes avant le test aux doses de 1, 2, 2,5, 4, 5 et 10 mg/kg. L'activité locomotrice (nombre de mouvements) est mesurée pendant 15 minutes.

Contrairement à la chlorpromazine, les composés se montrent peu ou non sédatifs (cf. Tableau III).

Tableau III

**Nombre de mouvements**

(en % par rapport à un lot témoin)

| DOSE IP | 1 mg/kg | 2 mg/kg | 2,5 mg/kg | 4 mg/kg | 5 mg/kg | 10 mg/kg |
|---|---|---|---|---|---|---|
| Exemple 1 | | | | | -20 % ns | -9 % ns |
| Exemple 7 | | | -27 % ns | | -43 %** | -63 % ** |
| Chlorpromazine | -20 % ns | -58 % ** | | -64 % ** | | |

ns indique $p > 0,05$

** indique $p < 0,01$

La différence observée entre chlorpromazine (très sédative dès 2 mg/kg alors que la dose efficace est de 4 mg/kg) et les composés étudiés tient à leur passage limité de la barrière hématoencéphalique comme le montre la détermination de la corticostéronémie après administration d'IL-1.

**Exemple 35 : Mesure de la corticostéronémie après administration d'IL-1**

Trente minutes après administration intrapéritonéale de la molécule à tester, les souris sont traitées par 1 µg d'IL-1 injecté par voie intrapéritonéale. La corticostéronémie est déterminée 2 heures après par une technique appropriée.

Un lot témoin reçoit dans les mêmes conditions une administration intrapéritonéale du composé, puis d'une solution saline. Sous l'influence de IL-1, la corticostéronémie augmente de façon très importante (contrôle : + 735 %). Les composés revendiqués ne modifient pas sensiblement le taux basal de corticostérone.

Après administration d'IL-1, les niveaux de la corticostéronémie ne sont pas non plus différents de celui observé chez les témoins. En revanche, la chlorpromazine (qui traverse la barrière hématoencéphalique), abaisse l'hypercorticostéronémie de l'ordre de 86 %.

**Tableau IV**

| **Hypercorticostéronémie après administration d'IL-1** (ng de corticostérone/ml de sérum) | | |
|---|---|---|
| Composé (5 mg/kg) | Hypercorticostéronémie après adm. IL-1 (ng/ml sérum) | Baisse de l'hypercorticostéronémie par rapport au contrôle |
| Exemple 1 | 389 | 11.8% |
| Exemple 2 | 417 | 5.4% |
| Exemple 7 | 389 | 11.8% |
| Exemple 8 | 356 | 19.3% |
| Exemple 10 | 440 | 0.2% |
| Chlorpromazine (4 mg/kg) | 63 | 85.7% |
| Contrôle | 441 | - |

**Exemple 36 : Effet hypotenseur**

Contrairement à la chlorpromazine, les composés sont dénués d'effet hypotenseur chez le rat.

Aucune hypotension n'est observée à 20 mg/kg par voie intra-péritonéale chez le rat pour les composés des exemples 1, 2, 7 et 8. Ceci n'est pas le cas de la chlorpromazine qui, à la même dose, entraîne une hypotension sévère dès la 15ème minute.

**Exemple 37 : Inhibition de l'activité phospholipase $PLA_2$ de porc**

En plus de leur effet sur le TNF, les molécules possèdent également une activité inhibitrice de la phospholipase $A_2$ ($PLA_2$). La PLA2 hydrolyse les phospholipides membranaires des cellules en libérant de l'acide arachidonique (AA). L'inhibition de cette enzyme entraîne une réduction des taux d'AA libre non estérifié à partir duquel sont produits des métabolites tels que les leucotriènes et les prostaglandines proinflammatoires.
L'activité $PLA_2$ est mesurée en prenant comme substrat des membranes d'*E.coli* marquées avec de l'acide oléique tritié. La $PLA_2$ pancréatique de porc est incubée avec la préparation pendant 30 minutes à 37°C. Après centrifugation, la radioactivité du surnageant est comptée. L'activité inhibitrice du composé (1mM) est exprimée par rapport au contrôle.

**Tableau VI**

| Composé | % Inhibition à 1 mM |
|---|---|
| Exemple 1 | 72 % |
| Exemple 7 | 97 % |
| Exemple 10 | 98 % |
| Chlorpromazine | 76 % |
| Mépacrine | 67 % |

**Revendications**

1. Composés de formule générale (I) :

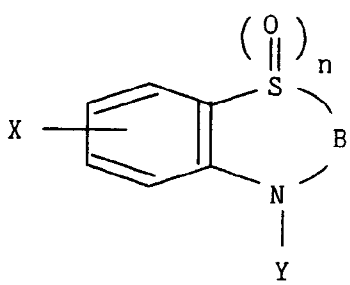

(I)

dans laquelle :
- X représente un atome d'halogène,
- n représente 0, 1 ou 2,
- B représente un des groupements $B_1$, $B_2$, $B_3$ de formules :

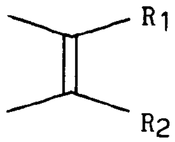

(B1)

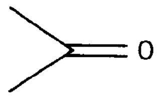

(B2)

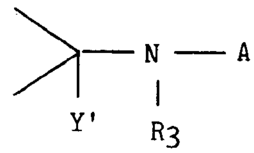

(B3)

dans lesquelles :

- $R_1$ et $R_2$ représentent indépendamment l'un de l'autre un atome d'hydrogène, un groupement alkyle éventuellement substitué et contenant de 1 à 5 atomes de carbone en chaîne droite ou ramifiée, un groupement aryle éventuellement substitué, un groupement arylalkyle éventuellement substitué, un groupement hétéroaryle éventuellement substitué,
  ou bien $R_1$ et $R_2$ forment ensemble un cycle aromatique contenant six atomes de carbone éventuellement substitué par un groupement X' représentant un atome d'halogène,
- $R_3$ représente un atome d'hydrogène, un groupement aryle éventuellement substitué, un groupement arylalkyle éventuellement substitué, un groupement hétéroaryle éventuellement substitué,
- A représente soit :
  * le radical

$$-(SO_2)_x- \text{ Alk - Z}$$

dans lequel x représente 0 ou 1, Alk représente un radical hydrocarboné saturé et bivalent, contenant de 3 à 6 atomes de carbone en chaîne droite ou ramifiée, et Z représente un radical choisi dans le groupe comprenant :

a) le radical nitrile, carboxy ou alcoxycarbonyle

b) le radical

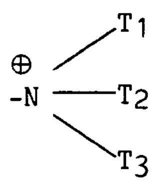

, Q⊖

dans lequel :

$T_1$, $T_2$, $T_3$, identiques ou différents, sont choisis parmi les radicaux alkyles linéaires ou ramifiés contenant de 1 à 5 atomes de carbone, les radicaux aryles éventuellement substitués et les radicaux arylalkyles éventuellement substitués,

Q représente un atome d'halogène, ou un résidu d'acide faible,

c) le radical

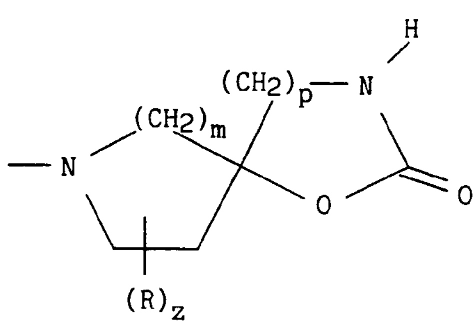

dans lequel m et p représentent indépendamment l'un de l'autre 1 ou 2, z représente un entier prenant les valeurs 0 à (m+2) (bornes incluses) et R représente un radical alkyle

contenant de 1 à 5 atomes de carbone en chaîne droite ou ramifiée, un radical aryle ou arylalkyle, éventuellement substitués,

d) le radical

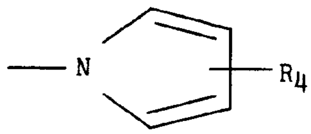

dans lequel $R_4$ représente un atome d'hydrogène, un radical carboxy, un radical alkoxy-carbonyle, ou un radical aminocarbonyle éventuellement substitué,

e) le radical

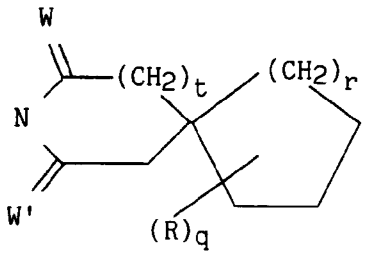

dans lequel W et W' représentent indépendamment l'un de l'autre $H_2$, O ou S, t représente 0 ou 1, r représente 1 ou 2, q représente un entier prenant les valeurs 0 à (r+2) (bornes incluses) et R a la même signification que précédemment,

f) le radical

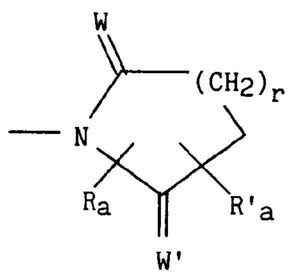

dans lequel W, W' et r sont tels que définis précédemment et $R_a$ et $R'_a$ représentent indépendamment l'un de l'autre un atome d'hydrogène, un radical alkyle contenant de 1 à 5 atomes de carbone en chaîne droite ou ramifiée, un radical aryle éventuellement substitué ou un radical arylalkyle éventuellement substitué,

g) le radical

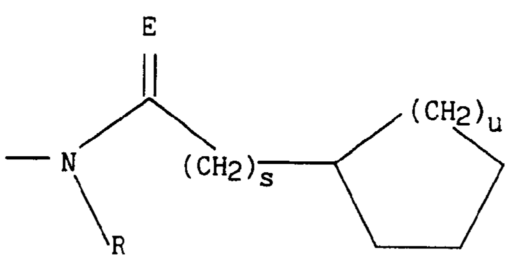

dans lequel E représente O, S ou le groupement N-H, s et u représentent indépendamment l'un de l'autre 1 ou 2 et R est tel que défini précédemment,

* soit le radical

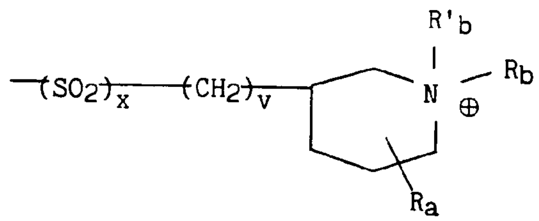

dans lequel v représente un entier prenant les valeurs 1 à 4 (bornes incluses) et $R_a$ et x sont tels que définis précédemment, et $R_b$ et $R'_b$ représentent indépendamment l'un de l'autre un radical alkyle contenant de 1 à 5 atomes de carbone en chaîne droite ou ramifiée, un radical aryle éventuellement substitué ou un radical arylalkyle éventuellement substitué,

* soit, lorsque B représente le radical $B_3$ précédemment défini, le radical

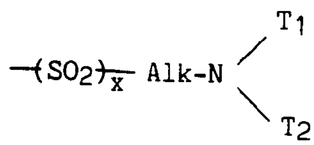

dans lequel x, Alk, $T_1$ et $T_2$ sont tels que définis précédemment,

* soit le radical

$$-(SO_2)_x-R_5$$

dans lequel x est tel que défini précédemment et $R_5$ représente un radical hydrocarboné contenant de 5 à 20 atomes de carbone en chaîne droite ou ramifiée et comprenant au moins une insaturation sous forme de double liaison,

- Y' forme avec Y une liaison,

- et Y, lorsque B représente les radicaux $B_1$ ou $B_2$ représente le groupement A tel que défini précédemment,

avec les restrictions suivantes :

- lorsque, simultanément B représente le groupement $B_2$, A représente le radical $-(SO_2)_x$Alk-Z t x est différent de 0, alors Z ne peut représenter ni un radical nitrile, ni un radical carboxy, ni un radical alcoxycarbonyle,
- lorsque simultanément B représente le radical $B_1$ dans lequel $R_1$ et $R_2$ forment ensemble un cycle aromatique à six carbones et A représente le radical

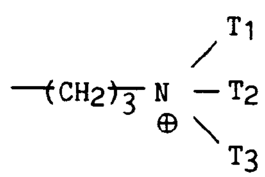 , $Q^\ominus$

alors $Q^\ominus$ ne peut représenter que l'anion $Br^\ominus$,

étant entendu que, sauf indication contraire, le terme "radical aryle" représente un radical choisi parmi phényle et naphtyle, que le terme "radical arylalkyle" représente un radical choisi parmi phényle et naphtyle fixé sur une chaîne alkyle comportant de 1 à 4 atomes de carbone, et que le terme "radical hétéroaryle" représente un radical choisi parmi pyridyle, pyrrolyle, pyrazinyle, pyridazinyle, pyrimidinyle, quinolyle et indolyle,

étant également entendu que, sauf indication contraire, l'expression "éventuellement substitué" signifie que les groupements alkyles, aryles, arylalkyles ou hétéroaryles peuvent être substitués par un ou plusieurs groupements choisis parmi les atomes d'halogène, les groupements hydroxy, nitro, cyano, alkyles, alkoxy, acyles, halogénoalkyles, amino, aminoalkyles et diaminoalkyles,

ainsi que leurs éventuels stéréoisomères, épimères, N-oxydes et sels d'addition à un acide ou à une base

pharmaceutiquement acceptables.

2. Composés selon la revendication 1 dans laquelle B représente le groupement $B_1$, leurs éventuels stéréoisomères, épimères, N-oxydes et sels d'addition à un acide ou à une base pharmaceutiquement acceptables.

3. Composés selon l'une quelconque des revendications 1 à 2 dans laquelle $R_1$ et $R_2$ forment ensemble un cycle aromatique non substitué contenant six atomes de carbone, leurs éventuels stéréoisomères, épimères, N-oxydes et sels d'addition à un acide ou à une base pharmaceutiquement acceptables.

4. Composés selon la revendication 3, dans laquelle A représente le groupement

$$-(SO_2)_x- \text{Alk-Z}$$

leurs éventuels stéréoisomères, épimères, N-oxydes et sels d'addition à un acide ou à une base pharmaceutiquement acceptables.

5. Composés selon la revendication 4 dans laquelle Z représente le radical

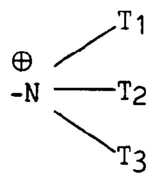 , $Q^{\ominus}$

leurs éventuels stéréoisomères, épimères, N-oxydes et sels d'addition à un acide ou à une base pharmaceutiquement acceptables.

6. Composés selon la revendication 4 dans laquelle Z représente le radical

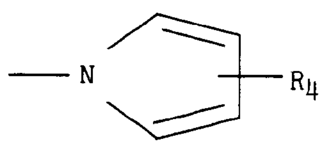

leurs éventuels stéréoisomères, épimères, N-oxydes et sels d'addition à un acide ou à une base pharmaceutiquement acceptables.

7. Composé selon l'une quelconque des revendications 1 à 5 qui est le bromure de N-[3-(2-chlorophénothiazin-10-yl)propyl]butyldiméthyl-ammonium, ainsi que ses sels d'addition à un acide pharmaceutiquement acceptables.

8. Composé selon l'une quelconque des revendications 1, 2, 3, 4 ou 6 qui est le 1-[3-(2-chlorophénothiazin-10-yl)-1-propyl]pyrrole-2-carboxylate de méthyle ainsi que ses sels d'addition à un acide pharmaceutiquement acceptables.

9. Procédé de préparation des composés de formule (I), caractérisé en ce que le produit de départ est :
a) - soit le composé de formule (II) :

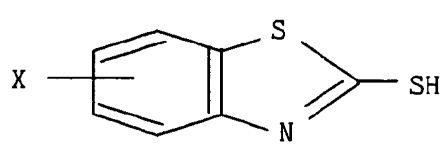 (II)

dans laquelle X est tel que défini pour la formule (I),
qui, traité par le chlorure de sulfuryle à température ambiante, conduit au composé de formule (III) :

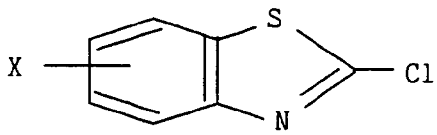

(III)

dans laquelle X est tel que défini précédemment,

puis par une amine de formule $H_2N-R_3$, dans laquelle $R_3$ est tel que défini précédemment pour donner le composé de formule (IV) :

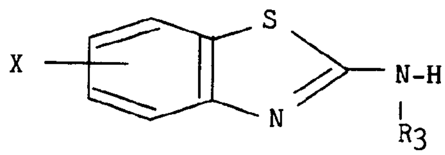

(IV)

dans laquelle X et $R_3$ sont tels que définis précédemment,

b)- soit le composé de formule (V) :

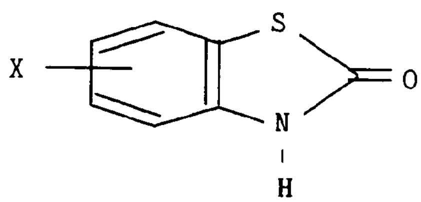

(V)

dans laquelle X est tel que défini précédemment,

c) - soit le composé de formule (VI) :

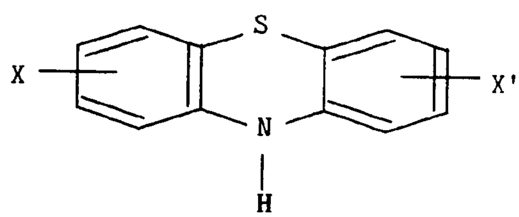

(VI)

dans laquelle X et X' sont tels que définis précédemment,

les composés de formules (IV), (V) ou (VI) formant l'ensemble des composés de formule (VII) :

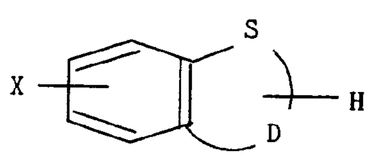

(VII)

dans laquelle X est tel que défini précédemment et D représente un des groupements $D_1$, $D_2$, ou $D_3$ de formules :

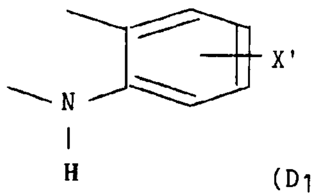

($D_1$)

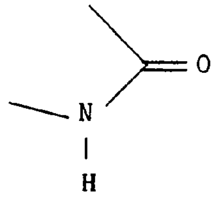

($D_2$)

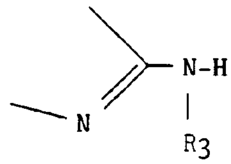

($D_3$)

dans lesquelles X' et $R_3$ sont tels que définis précédemment, que l'on oxyde éventuellement :

- en S-monoxyde au moyen d'un réactif approprié, suivi d'une neutralisation par une solution alcaline,
- en S-dioxyde, après protection éventuelle de l'atome d'azote, avec un agent oxydant approprié,

de manière à obtenir les composés de formule (VIII) :

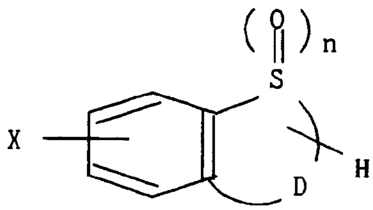

(VIII)

où X, n et D sont tels que définis précédemment,
qui sont soumis :
a) soit à une alkylation, à l'aide d'amidure de sodium avec le composé de formule (IX) :

$$Hal-(SO_2)_x-R_5 \quad (IX)$$

dans laquelle x et $R_5$ sont tels que précédemment définis et Hal représente un atome d'halogène, pour obtenir les composés de formule (X) :

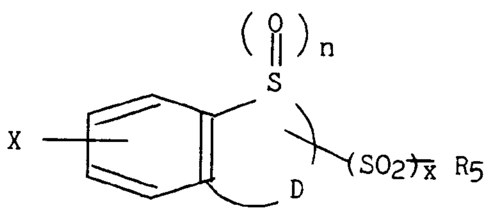

(X)

dans laquelle X, n, D, x et $R_5$ sont tels que définis plus haut,
b) soit à une alkylation, à l'aide d'amidure de sodium en solvant approprié, avec un composé de formule (XI) :

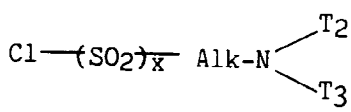

(XI)

dans laquelle x, Alk, $T_2$, et $T_3$ sont tels que définis précédemment,
pour donner le composé de formule (XII) :

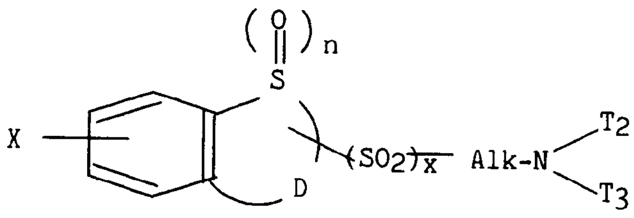

(XII)

où X, n, D, x, Alk, $T_2$ et $T_3$ ont les mêmes définitions que précédemment,
qui sera éventuellement transformé par le composé de formule (XIII) :

$T_1$-Q (XIII)

où $T_1$ et Q sont tels que définis précédemment,
en sel d'ammonium quaternaire de formule (XIV) :

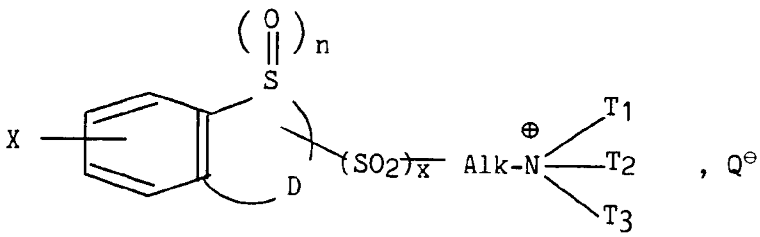

(XIV)

où X, n, D, x, Alk, $T_1$, $T_2$, $T_3$ et Q sont tels que définis précédemment,
c) soit à une alkylation, dans un mélange cétone / base, avec le composé de formule (XV) :

$J \text{-}(SO_2)_x\text{-} Alk\text{-}Cl$ (XV)

dans laquelle x et Alk sont tels que définis précédemment et J représente un atome de chlore ou de brome,
conduisant au synthon de formule (XVI) :

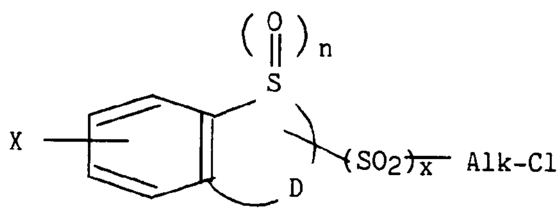

(XVI)

dans laquelle X, n, D, x et Alk sont tels que définis précédemment,
qui traité par :

α) le cyanure de tétraméthylammonium en présence d'alcool, conduit au composé de formule (XVII) :

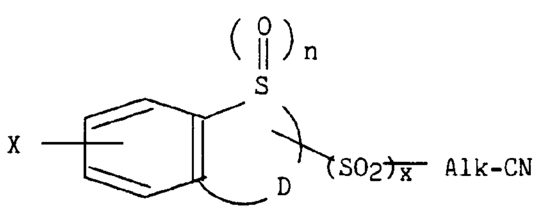

(XVII)

dans laquelle X, n, D, x et Alk sont tels que définis précédemment,

qui, par hydrolyse éventuelle, donne le composé de formule (XVIII) :

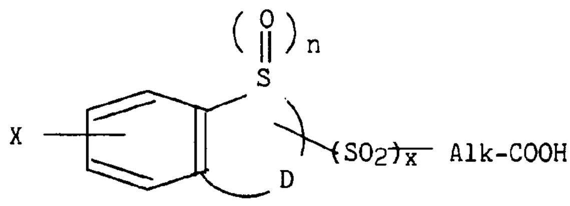

(XVIII)

dans laquelle X, n, D, x et Alk sont tels que définis précédemment,
qui peut être transformé en esters ou en amides correspondants selon les méthodes classiques,
β) un des composés suivants :

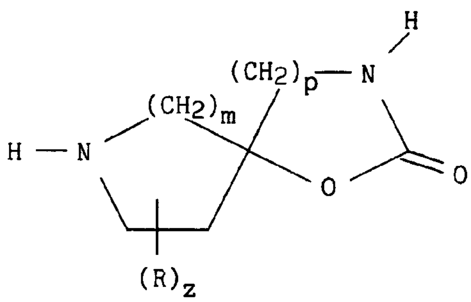

;

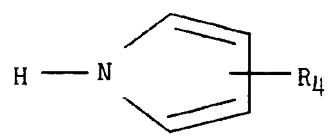

;

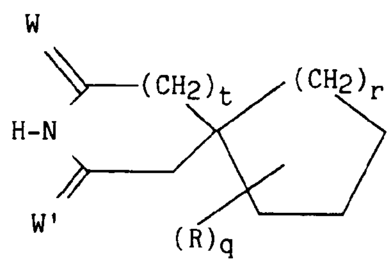

;

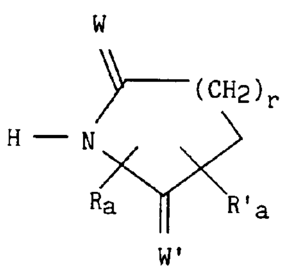

;

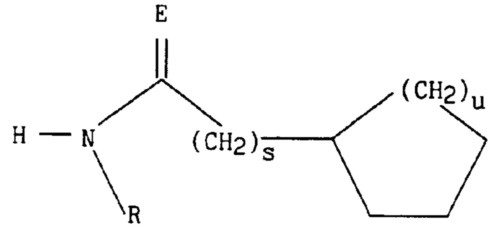

pour lesquels m, p, q, r, s, t, u, z, R, $R_a$, $R'_a$, $R_4$, E, W et W' sont tels que définis précédemment,
en présence d'un sel alcalin, d'une base alcaline et d'éther couronne, conduit respectivement aux composés de formules (XIXa) à (XIXe) suivantes :

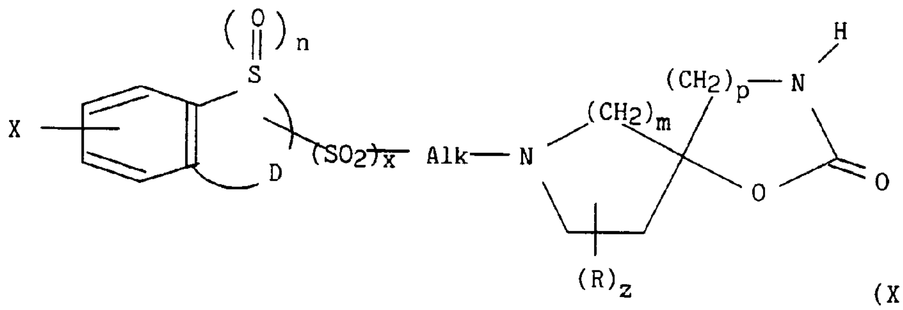

(XIXa)

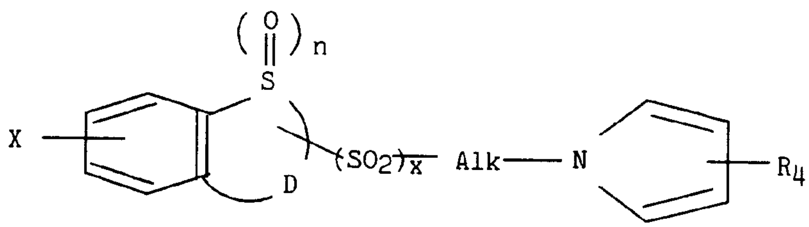

(XIXb)

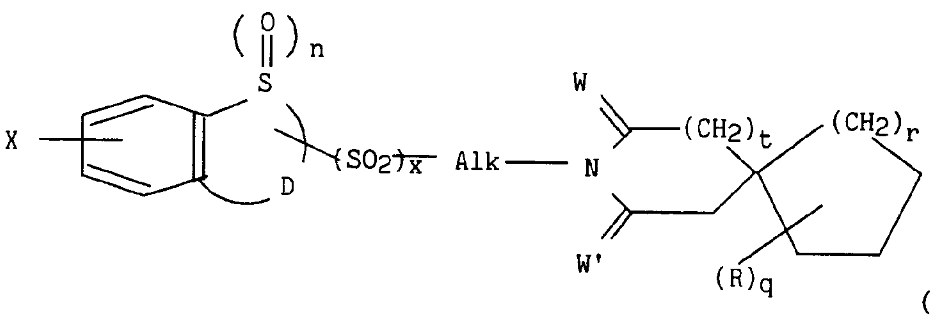

(XIXc)

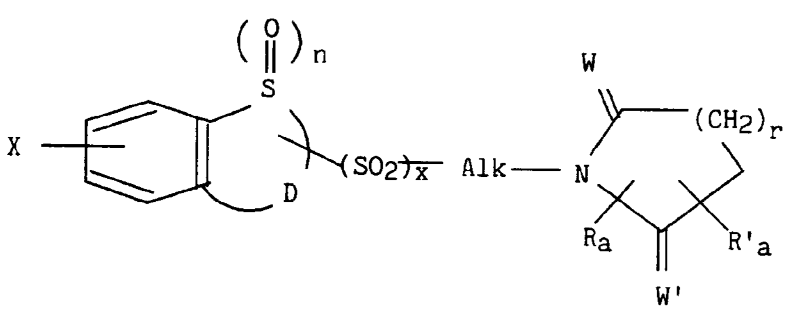

(XIXd)

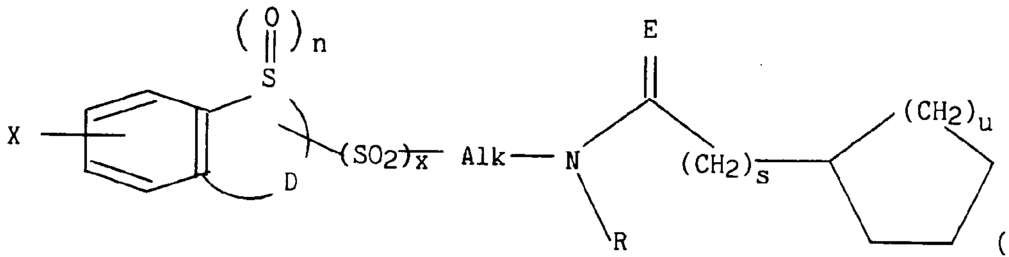

(XIXe)

dans lesquelles m, n, p, q, r, s, t, u, x, z, Alk, D, E, R, $R_a$, $R'_a$, $R_4$, W, W' et X sont tels que définis précédemment,

d) soit à une alkylation, dans un mélange cétone / base, avec le composé de formule (XX) :

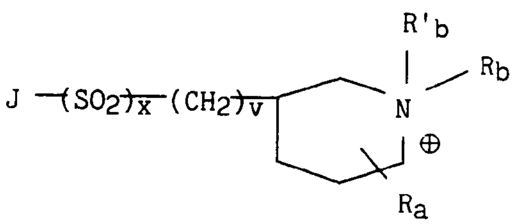

(XX)

dans laquelle J, $R_a$, $R_b$, $R'_b$, x et v sont tels que définis précédemment,
conduisant au composé de formule (XXI) :

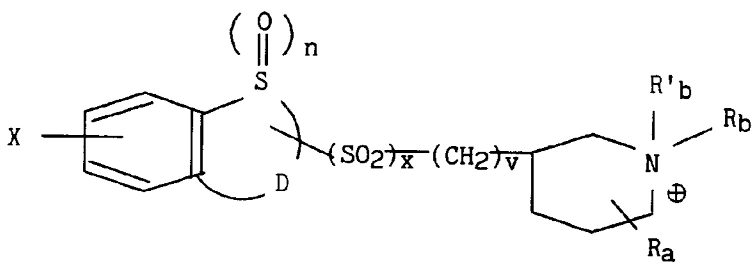

(XXI)

dans laquelle X, n, D, x, v, $R_a$, $R_b$ et $R'_b$ sont tels que définis précédemment,

les composés de formules (X), (XII), (XVII), (XIXa) à (XIXe) ou (XX) lorsque D représente le groupement

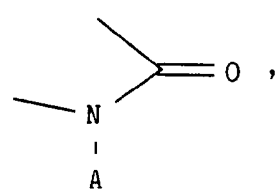

dans lequel A est tel défini précédement, pouvant être soumis à l'action d'une base dans un solvant alcool, puis acidification, pour conduire au composé de formule (XXII) :

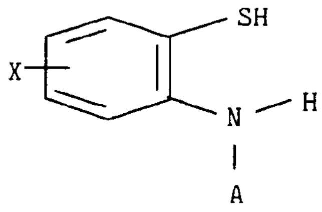

(XXII)

dans laquelle X et A sont tels que définis précédemment, qui, traité en milieu alcalin, par un composé de formule (XXIII) :

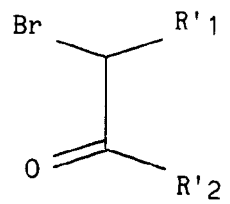

(XXIII)

dans lequel $R'_1$ et $R'_2$ représentent indépendamment l'un de l'autre un atome d'hydrogène, un groupement alkyle éventuellement substitué et contenant de 1 à 5 atomes de carbone en chaîne droite ou ramifiée, un groupement aryle éventuellement substitué ou un groupement hétéroaryle éventuellement substitué,
conduit au composé de formule (XXIV) :

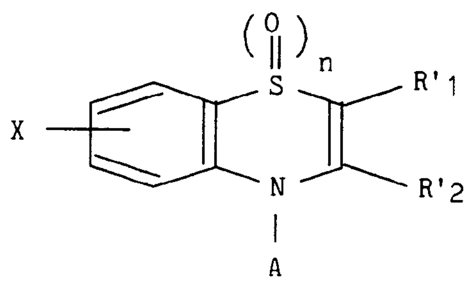

(XXIV)

dans laquelle X, A, n, $R'_1$ et $R'_2$ sont tels que définis précédemment,
l'ensemble des composés de formules (X), (XII), (XIV), (XVII), (XVIII), (XIXa) à (XIXe), (XX), (XXI) et (XXIV) formant l'ensemble des composés de formule (I) que l'on purifie le cas échéant selon une technique classique de purification et dont on sépare, si on le souhaite, les stéréoisomères par une technique classique de séparation et que l'on transforme, si nécessaire, en leurs sels d'addition à un acide ou une base pharmaceutiquement acceptables, composés qui, lorsque n = 0 peuvent être oxydés en S-oxyde ou S-dioxyde correspondants.

**10.** Composition pharmaceutique contenant comme principe actif au moins un composé selon l'une des revendications 1 à 8 seul ou en combinaison avec un ou plusieurs excipients ou véhicules inertes, non toxiques, pharmaceutiquement acceptables.

**11.** Composition pharmaceutique selon la revendication 10 contenant au moins un principe actif selon l'une des revendications 1 à 8 possédant une activité inhibitrice vis-à-vis des cytokines et utiles dans le traitement de la douleur, des rejets de greffons des affections inflammatoires et des affections pouvant induire un choc septique.